# EUROPÄISCHE PATENTSCHRIFT

(11) **EP 0 689 448 B1**
(45) Veröffentlichungstag und Bekanntmachung des Hinweises auf die Patenterteilung: **03.12.1997**
(21) Anmeldenummer: 94910356.8
(22) Anmeldetag: 23.03.1994
(51) Int. Cl.: A61K 38/48

(54) **ANGIOGENIN UND/ODER ALPHA1-MICROGLOBULIN UND/ODER KOMPLEMENTFAKTOR D ZUR BEGRENZUNG DER SEKRETION VON PROTEINEN**
ANGIOGENINE, AND/OR ALPHA1-MICROGLOBULINE AND/OR COMPLEMENTARY FACTOR D FOR LIMITING PROTEIN SECRETION
ANGIOGENINE, ET/OU DE ALPHA1-MICROGLOBULINE ET/OU DU FACTEUR COMPLEMENTAIRE D'AFIN DE LIMITER LA SECRETION DE PROTEINES

(30) Priorität: 23.03.1993 DE 4309391
(43) Veröffentlichungstag der Anmeldung: 03.01.1996
(73) Patentinhaber: Tschesche, Harald, D-33615 Bielefeld (DE)
(72) Erfinder: TSCHESCHE, Harald, D-33615 Bielefeld (DE); HEMPELMANN, Ute, D-40670 Meerbusch (DE)
(74) Vertreter: Pfenning, Meinig & Partner
(86) Internationale Anmeldenummer: DE9400333
(87) Internationale Veröffentlichungsnummer: WO9421273

(56) Entgegenhaltungen:
- EP-A- 0 255 011
- WO-A-86/06079
- THE JOURNAL OF BIOLOGICAL CHEMISTRY, Bd.267, Nr.30, 25. Oktober 1992, BALTIMORE MD, VSA Seiten 21982 - 21986 S. SAXENA ET AL. 'Angiogenin is a cytotoxic, tRNA-specific ribonuclease in the RNase A superfamily.'
- BRITISH JOURNAL OF CANCER, Bd.57, Nr.6, Juni 1988, LONDON, GROSSBRITANNIEN Seiten 587 - 590 J. RIORDAN ET AL. 'Human angiogenin, an organogenic protein.'
- BIOCHEMISTRY, Bd.27, Nr.19, 20. September 1988, WASHINGTON DC, VSA Seiten 7263 - 7268 D. ST.CLAIR ET AL. 'Angiogenin abolishes cell-free protein synthesis by specific ribonucleolytic inactivation of 40S ribosomes.'
- SCIENCE, Bd.235, 23. Januar 1987, WASHINGTON DC, VSA Seiten 442 - 447 J. FOLKMAN ET AL. 'Angiogenic factors.'

## Beschreibung

Verwendung von Angiogenin und/oder α₁-Microglobulin und/oder Komplementfaktor D zur Herstellung eines Therapeutischen Mittels zur Begrenzung der Sekretion von Enzymen aus Leukozyten und Therapeutisches Mittel hierzu.

Die Erfindung betrifft die Wirkung von Angiogenin und/oder α₁-Microglobulin und oder Komplementfaktor D zur Herstellung eines Therapeutischen Mittels zur Begrenzung bzw. Verhinderung der Sekretion von Enzymen aus Leukozyten. Die Erfindung betrifft weiterhin ein therapeutisches Mittel für die entsprechende Verwendung.

Leukozyten, speziell Granulozyten, sezernieren insbesondere nach Stimulierung durch unphysiologische Oberflächen, durch Chemotaxine, wie Formylpeptide, Komplementkomponenten, Leukotriene u.a., Enzyme, speziell Proteasen in ihre Umgebung. Diese Enzyme (Kollagenasen und Gelatinasen) dienen dem Durchtritt der Zellen durch das Gewebe, dem Abräumen zerstörten Gewebes bei der Wundheilung und dem Abbau eingedrungener Fremdkörper. Die proteolytischen Enzyme (Kollagenase, Gelatinase, Elastase, Cathepsin G und B und andere) werden in ihrer lokalen Wirkung normalerweise durch Inhibitoren (TIMP-1, TIMP-2, α-Proteinaseinhibitor, α₁-Antichymotrypsin, Cystatine, α₁-Makroglobulin u.a.) des Gewebes, Serums und Blutplasmas rasch gehemmt.

Im Fall einer massiven Leukozyteninvasion wie bei Entzündungsreaktionen kann es zu einem Zustand kommen, in dem das lokale Inhibitorpotential nicht ausreicht und das überschießende Enzympotential zu erheblichen Schädigungen des körpereigenen Gewebes führt. Krankheitsbilder mit derartigen entzündlichen Entgleisungen sind z.B. das Lungenemphysem, die entzündlich rheumatoide Arthritis, Lupus erythematodes u.a. Aber auch die Hämodialyse bei Patienten mit chronischer Niereninsuffizienz führt im Dialysator an der Oberfläche der Dialysemembran zu einer massiven Aktivierung der Leukozyten mit entsprechender Freisetzung der genannten Enzyme. Gleiches erfolgt bei der extrakorporalen Zirkulation bei Operationen (z.B. am offenen Herzen). Die eingesetzten Enzyme führen dann zu einer erheblichen (bis zu 50%) proteolytischen Zerstörung wichtiger Blutplasmaproteine (α₁-Proteinaseinhibitor, α₁-Antichymotrypsin, C₁-Esteraseinhibitor, u.a.). Der bisherige Therapieansatz bei derartigen, entzündlich degenerativen Erkrankungen, z.B. Lungenemphysem, konzentrierte sich auf die Wiederherstellung der Enzym/Inhibitorbalance durch therapeutische Verstärkung des Inhibitorpotentials, z.B. Infusion entsprechender Enzyminhibitoren, [Lang, H. und Greiling, H. (Hrsg.) Pathobiochemie der Entzündung, Springer Verlag, Berlin (1983)].

Ausgehend hiervon ist es die Aufgabe der vorliegenden Erfindung ein Verfahren zur Herstellung eines therapeutischen Mittels anzugeben daß bekannte und/oder neue Wirkstoffe enthält, die die Sekretion von Enzymen aus Leukozyten verhindern oder zumindest stark begrenzen und entsprechende Therapeutische Mittel vorzuschlagen.

Die Lösung dieser Aufgabe wird dadurch erreicht, daß erstmalig vorgeschlagen wird, zur Verhinderung oder Begrenzung der Sekretion von Proteinen, Stoffe mit sekretionshemmender Wirkung einzusetzen. Solche Stoffe wurden erstmals als Blutplasmaproteine aufgefunden und mit ihrer neuartigen Wirkungsweise identifiziert.

Hierzu gehören Angiogenin und/oder α₁-Microglobulin und/oder Komplementfaktor D bzw. deren Fragmente bzw. Peptide mit den biologisch wirksamen Sequenzbereichen dieser Peptide. Die schutzhemmende Wirkung zeigt sich besonders deutlich an den Inhibitionskurven 2 bis 4 für die drei Proteine. Gleichzeitig wird ein neues therapeutisches Mittel, wie es in den Ansprüchen 2 bis 7 gekennzeichnet ist, vorgeschlagen. Die Unteransprüche zeigen vorteilhafte Weiterbildungen auf.

Überraschenderweise wurde festgestellt, daß durch die Verwendung von Angiogenin und/oder α₁-Microglobulin und/oder Komplementfaktor D und/oder deren Fragmenten bzw. Peptiden mit den biologisch wirksamen Sequenzbereichen dieser Peptide, die Ausschüttung der Enzyme aus den Leukozyten auch in Gegenwart von Stimulantien unterdrückt wird. Die vorstehend erwähnten Wirkstoffe sind dabei äußerst wirksam und unterdrücken bereits in nanomolaren Konzentrationen im Medium die Ausschüttung von Proteinen inkl. Enzymen aus stimulierten und unstimulierten (Spontanfreisetzung) Zellen.

Erfindungsgemäß werden unter Peptidomimetika chemische Substanzen verstanden, deren Struktur sich von Peptiden herleitet und in denen bestimmte Strukturbereiche durch andere chemische Strukturen ersetzt sind, wobei die biologischen Eigenschaften des ursprünglichen Peptides erhalten oder potenziert werden. Peptidomimetika sind zum Beispiel peptidanaloge Verbindungen, in denen die Peptidbindung -CO-NH- ersetzt ist durch beispielsweise -CO-CH₂-, -CH₂-CH₂-, -CH₂-NH- und andere Gruppen oder in denen Seitenketten der natürlichen, proteinogenen Aminosäuren ersetzt sind durch andere chemische Strukturen oder aber Verbindungen, in denen beides erfolgt ist, aber die biologische Wirkung erhalten geblieben ist.

Die Anmelderin konnte dabei überraschenderweise zeigen, daß die vorstehend erwähnten Wirkstoffe sowohl einzeln wie auch in Kombination die erfindungsgemäße Verwendung zeigen.

Bevorzugterweise wird vorgeschlagen, Angiogenin und/oder deren Fragmente bzw. Peptide zu verwenden.

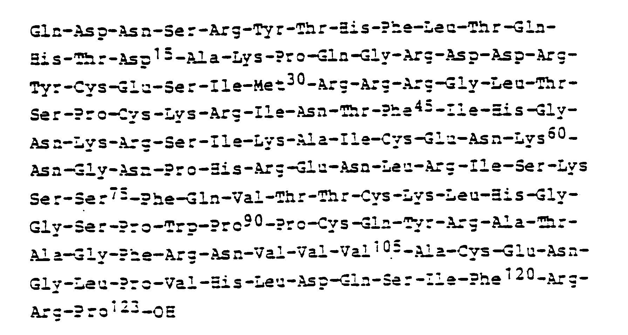

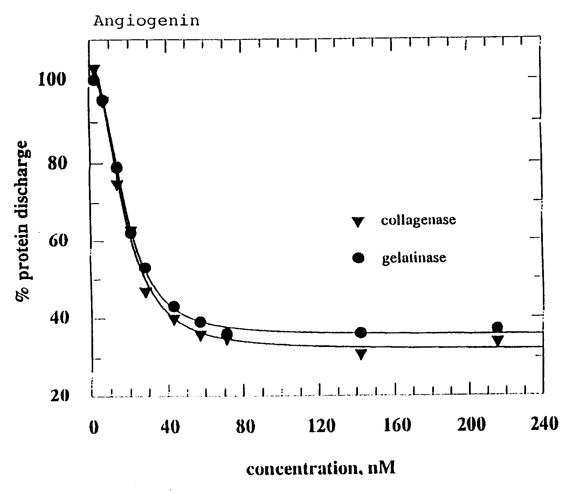

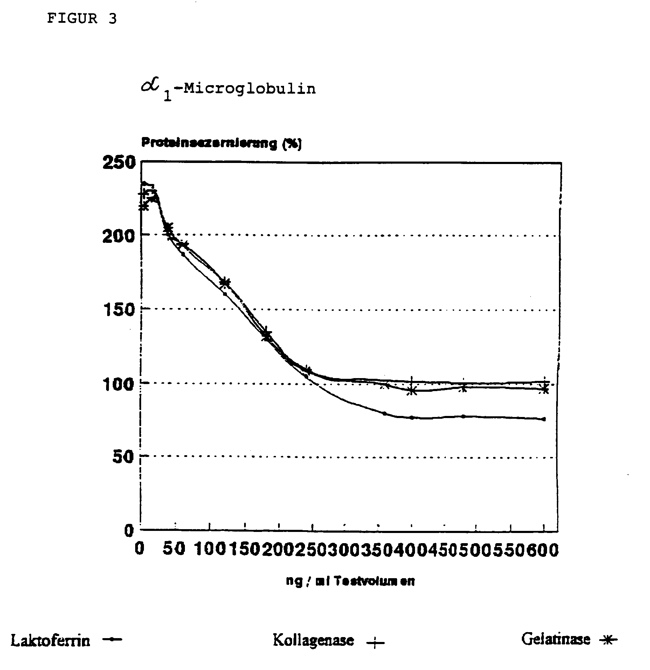

Es wurde dabei gefunden, daß bei der Verwendung von Angiogenin außer der Proteinsekretion alle anderen anti-inflammatorischen Funktionen wie Chemotaxis, Respiratory Burst und Phagozytose nicht nachhaltig beeinflußt werden. Bei dem erfindungsgemäß eingesetzten Angiogenin (Fig. 1) handelt es sich um humanes Angiogenin mit einem Molekulargewicht von Mᵣ 14.400, das als Angiogenesefaktor in geringer Konzentration in normalem, menschlichen Blutplasma vorkommt [R. Shapiro, D. J. Strydon, K. A. Olson und B. L. Vallee, Biochemistry 26, 5141-5146 (1987)]. Das isolierte Protein ist hinsichtlich Molekulargewicht, Immunreaktivität, chromatographischem Verhalten, Aminosäureanalyse und Sequenz identisch mit Angiogenin. Authentisches, rekombinantes Angiogenin [K. Kurachi, E. W. Davie, D. J. Strydon, J. F. Riordan and B. L. Vallee, Biochemistry 24, 5494-5499 (1985)] zeigt im Degranulations-Hemmtest an Granulozyten den gleichen Effekt wie das isolierte Protein.

Angiogenin bindet rasch an die Plasma-Membran von verschiedenen Zellinien bei 37°C (t_{1/2}< 1 min), aber in vergleichsweise kleinen Mengen. Es ist kein direktes Mitogen, aber es kann die primäre mitogene Stimulierung bei verschiedenen Zellinien modulieren, wobei dies korreliert mit der in vitro-Synthese von Phosphatidylinositol-biphosphat (PtdInsP₂). PtdInsP₂ hat eine entscheidende Mittlerrolle in der intrazellulären Signaltransduktion [W.F. Heath, Jr., F. Moore, R. Bicknell und B.L. Vallee, Proc. Natl. Acad. Sci. USA 86, 2718-2722 (1989)]. Angiogenin induziert die Vaskularisation in chorioallantoiner Membran von Hühnern und in Kaninchen Cornea [J.W. Fett, D.J. Strydon, R.R. Lobb, E.M. Alderman, H.L. Bethune, J.F. Riordan und B.L. Vallee, Biochemistry 24, 5480-5486 (1985)] und aktiviert die zelluläre Phospholipase C, was zu einem vorübergehenden Anstieg intrazellulären 1,2-Diacylglycerols aus der Hydrolyse von Phosphatidylinositol führt [R. Bicknell und B.L. Vallee, Proc. Natl. Acad. Sci. USA 85, 5961-5965 (1988)].

Überraschenderweise hat sich gezeigt, daß Angiogenin die Spontan-Degranulation polymorphkerniger Leukozyten (PMNL) um 60% und mehr hemmt. Die mit Angiogenin vorinkubierten Zellen reagieren auf Stimulierung mit FNLPNTL (Formyl-Norleucyl-Leucyl-Phenylalanyl-Norleucyl-Tyrosyl-Leucin) nicht mehr mit Degranulation. Die sezernierte Proteinmenge liegt in diesem Fall 40% unter dem Wert für die unbehandelten Kontrollen. Mehrfaches Waschen dieser Zellen mit Puffer stellt die Sensitivität gegenüber dem Stimulans FNLPNTL nicht wieder her.

Die biologische Aktivität der Degranulation ist konzentrationsabhängig. 0,1 µg/ml werden mindestens benötig, um eine minimale Hemmung der Degranulation hervorzurufen. Unterhalb dieser Angiogenin-Konzentration kann kein Hemmeffekt beobachtet werden. Maximale Hemmung wird mit 1 µg/ml erreicht. Ein Hemmeffekt wird aber auch noch bei einer Konzentration von 2 µg/ml erreicht. Das Mittel muß dabei den Wirkstoff in einer Konzentration enthalten, so daß die angegebene Konzentration im Blut erreicht wird.

Die Hemmung der Degranulation von PMNL ist nicht auf eine Zerstörung der für die Proteinsekretion erforderlichen Mikrotubuli durch Angiogenin zurückzuführen. Auch eine kompetitive Besetzung der FNLPNTL-Rezeptoren durch Angiogenin ist als Ursache für den Hemmeffekt auszuschließen, da die Zellen noch zur Chemotaxis entlang eines FNLPNTL-Gradienten befähigt sind.

Die Wirkung von Angiogenin tritt innerhalb von Sekunden ein. Die Zellen reagieren auf das Agens in einer Zeitspanne, die mit der Reaktion auf das chemotaktische Peptid FNLPNTL vergleichbar ist.

Überraschend ist besonders, daß Angiogenin in den genannten Konzentrationen keinen Einfluß auf die Chemotaxis, auf die Phagozytose und auf den "oxidative burst" von PMNL hat. Es handelt sich also nicht um eine generelle "Lähmung" der Zelle oder ihrer übrigen biologischen Abwehrfunktionen.

Der Degranulation inhibierende Effekt wird auch von proteolytisch gewonnenen Fragmenten des Angiogenins in gleicher molarer Konzentration bewirkt. So haben einzelne isolierte tryptische Fragmente die gleiche, wenn auch etwas verminderte Wirkung auf Granulozyten wie das intakte Angiogenin. Besonders bevorzugt ist hierbei das Fragment mit der folgenden Sequenz, wobei die Reste 34-40 mit den Resten 83-95 über Cys verbrückt sind:

Gly³⁴-Leu-Thr-Ser-Pro-Cys-Lys⁴⁰
Leu⁸³-His-Gly-Gly-Ser-Pro-Trp-Pro-Pro-Cys-Glu-Tyr-Arg⁹⁵

Geeignet sind auch Derivate mit entsprechender Wirkung. Bevorzugt ist hierbei ein Fragment, das aus der Aminosäuresequenz Leu⁸³ - Arg⁹⁵ besteht, mit einem freien Cys an der Position 92. Ähnliche Eigenschaften werden auch mit verkürzten Sequenzen, d.h. mit Sequenzen erzielt, bei denen noch weitere Aminosäuren der Kette Leu⁸³- Arg⁹⁵ abgespalten sind.

Die Hemmung ist allerdings nicht ganz so stark, verglichen mit intaktem Angiogenin.

Es ist weiterhin bevorzugt, wenn die Disulfide der vorstehend beschriebenen Fragmente eingesetzt werden. Die synthetisch hergestellten gemischten Disulfide zeigen dabei die gleiche Wirkung wie das isolierte Fragment.

Die Erfindung schließt aber auch alle anderen Fragmente mit ein, insoweit sie ebenfalls die Hemmung der Sekretion von Proteinen bewirken.

Die erfindungsgemäße Verwendung wird auch von Mischungen von komplettem Angiogenin und Fragmenten mit den biologisch wirksamen Sequenzbereichen erreicht.

Auch synthetische Peptide sind geeignet. Diese Peptide können entweder allein als Wirkstoff oder in Kombination mit anderen Fragmenten oder in Kombination mit dem kompletten Angiogenin vorliegen.

Ein derartiges synthetisches Peptid, das ebenfalls ausgezeichnete Hemmeigenschaften hat, enthält die Reste 83 bis 95 mit Ser⁹² anstelle Cys⁹². Dieses Peptid besitzt noch ca. 50% der Hemmwirkung verglichen mit intaktem Angiogenin. Die Erfindung umfaßt auch hier verkürzte Varianten davon.

Weiterhin ist es bevorzugt, α₁-Microglobulin zu verwenden. α₁-Microglobulin ist ein heterogen geladenes Glykoprotein mit einem Molekulargewicht von ca. Mᵣ 30.000, das in den Harn ausgeschieden wird (B. Berggärd, B. Ekström und B. Äkerström, Scand. J. Clin. Lab. Invest. 40, Suppl. 154, 63-71, 1980). Die Identifizierung erfolgte über Aminosäureanalyse und Analyse der Aminosäuresequenzen. α₁-Microglobulin ist mit ^{∼} 0,03 mg/ml eine Plasmakomponente, wobei es zur Hälfte in einem 1:1 Komplex mit IgA vorliegt. (A. Grubb, E. Mendez, J.L. Fernandez-Luna, C. Lopez, E. Mihaesco und J.P. Vaerman, J. Biol. Chem. 261, 14313-14320, 1986). Es wurde außerdem in Zellen der Monozyten-Makrophagenlinie in Thymus, Milz, Lymphknoten, Mandeln, Hepatozyten und Drüsen des Verdauungstraktes nachgewiesen (P. Bonic, C. Vincent und J.P. Revillard, Histochem. J. 16, 1311-1324, 1984). Aufgrund der Aminosäuresequenz kann das Protein der Lipocalin-Superfamilie der Plasmaproteine zugeordnet werden (S. Perviaz und K. Brew, Science 228, 335-337, 1985).

Das Immunsystem wird durch α₁-Microglobulin in dreifacher Hinsicht beeinflußt. Die in Vitro-Stimulierung von Lymphozyten durch verschiedene Proteinantigene wird gehemmt. Dieser Effekt wird auch durch Glykopeptide des Proteines hervorgerufen, was auf eine geringe Bedeutung des Peptidrückgrats hinweist (B. Äkerström und L. Lögdberg, Scand. J. Immunol. 20, 559-563, 1984). α₁-Microglobulin steigert den background ³H-Thymidineinbau in T-Zellen und B-Zellen, der unter strikter Regulation durch andere Serumkomponenten steht (H. Babiker-Mohamed, B. Äkerström und L. Lögdberg, Scand. J. Immunol. [in press, 1990]). Die in vitro Migration neutrophiler Granulozyten wird durch α₁-Microglobulinkonzentrationen oberhalb 0,6 mg/ml inhibiert (E. Mendez, J.L. Fernandez-Luna, A. Grubb und F. Levya-Cobian, Proc. Natl. Acad. Sci USA 83, 1472-1475, 1986) von geringen Konzentrationen aber nicht beeinflußt. Das Protein scheint eine bisher noch nicht geklärte physiologische Funktion bei entzündlichen Prozessen zu haben.

Auf einer menschlichen Zellinie wurde ein Rezeptor für α₁-Microglobulin beschrieben (J.L. Fernandez-Luna, F. Levya-Cobian und F. Mollinedo, FEBS Lett. 236, 471-474, 1988), der nach Zugabe von PMA zum Kulturmedium exprimiert wird.

Überraschenderweise hat sich gezeigt, daß α₁-Microglobulin die Spontan-Degranulation um 40 % und mehr hemmt. Wird die Spontan-Degranulation als Kontrollwert gleich 100% gesetzt, dann steigert die Stimulierung durch FNLPNTL die Degranulation auf 230%. Mit α₁-Microglobulin behandelte Zellen zeigen dagegen eine Hemmung der Proteinausschüttung, die 10 -15% unter dem Wert der Spontan-Degranulation, d.h. nur bei 85% liegt.

Die Hemmaktivität ist konzentrationsabhängig. Eine minimale Hemmung wird mit 0,1µg α₁-Microglobulin/ml erreicht. Niedrigere Konzentrationen rufen keinen nennenswerten Hemmeffekt hervor. Maximale Hemmung der Degranulation ist mit 0,2µg/ml erreichbar. Die beobachtete Hemmung ist aus den bereits für Angiogenin beschriebenen Gründen ebenfalls nicht auf eine kompetitive Besetzung der FNLPNTL-Rezeptoren durch α₁-Microglobulin zurückzuführen. Es konnte gezeigt werden, daß die Microtubuli nicht zerstört werden.

Die Erfindung schließt auch biologisch wirksame Sequenzbereiche von α₁-Microglobulin ein, insoweit sie eine Hemmung bzw. Verhinderung der Sekretion von Proteinen bewirken.

Die Anmelderin konnte zeigen, daß neben den beiden vorstehenden Wirkstoffen, nämlich dem Angiogenin und dem α₁-Microglobulin auch der Komplementfaktor D in gleicher Weise für die erfindungsgemäße Verwendung geeignet ist. Wie bereits eingangs ausgeführt ist es hierbei möglich, daß die einzelnen Wirkstoffe entweder alleine oder auch in Kombination untereinander oder nur in Kombination mit einzelnen Wirkstoffen eingesetzt werden. Es hat sich dabei gezeigt, daß hierbei zum Teil additive Effekte auftreten, wenn die normale Hemmung noch nicht erreicht ist.

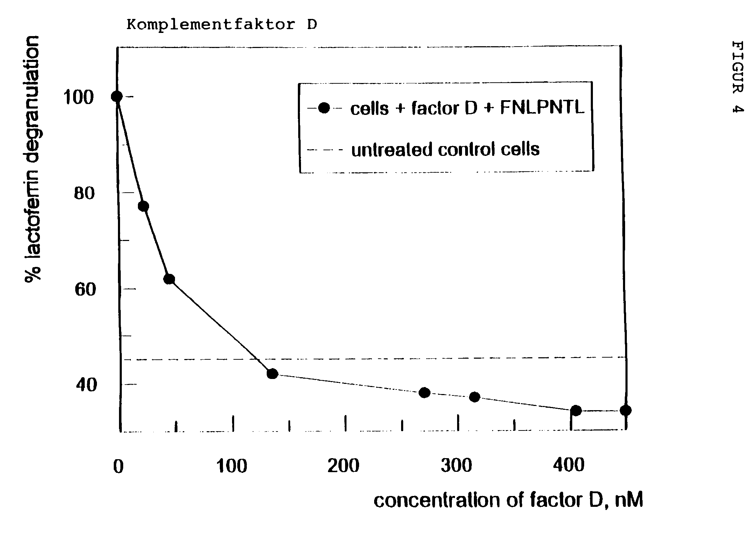

Komplementfaktor D ist identisch mit dem humanen Adipsin (White, R.T., Damm, D., Hancock, N., Rosen, B.S., Lowell, B.B., Usher, P.,Flier, J.S. and Spiegelman, B.M. (1992) J. Biol. Chem. 267, 9210-9213). Komplementfaktor D hat ein Molekulargewicht von 24 kD und enthält eine Polypeptidkette. Die Anmelderin konnte nun zeigen, daß auch der Komplementfaktor D die Spontandegranulation polymorphkerniger Leukozyten hemmt. Fig. 2 zeigt hierbei die Wirkung von Komplementfaktor D am Beispiel der Lactoferrin-Degranulation. Es wurde dabei gefunden, daß unbehandelte Zellen 45 % Lactoferrin sekretieren, wohingegen mit Komplementfaktor D der aus Fig. 2 ersichtliche Effekt eintritt.

Die Erfindung betrifft weiterhin ein therapeutisches Mittel zur Begrenzung bzw. Verhinderung der Sekretion von Proteinen. Erfindungsgemäß wird vorgeschlagen, daß das Mittel entweder α₁-Microglobulin oder Komplementfaktor D enthält. Bevorzugt ist es dabei, daß das therapeutische Mittel die vorstehend beschriebenen Wirkstoffe Angiogenin, α₁-Microglobulin und Komplementfaktor D entweder in Kombination von allen drei Wirkstoffen oder jeweils in Kombination von zwei Wirkstoffen enthält. Das therapeutische Mittel enthält dann noch üblicherweise die an und für sich bekannten Träger und Zusatzstoffe. Besonders bevorzugt ist dabei, wenn das therapeutische Mittel die bereits vorstehend erwähnten Fragmente von Angiogenin enthält.

Das erfindungsgemäße Mittel kann entweder in Form einer Lösung, z.B. für die intravenöse Behandlung, als Spray oder als feste Formulierung verabreicht werden.

Bevorzugterweise enthält das Mittel den Wirkstoff in einer derartigen Konzentration, daß im Blut eine Konzentration von 0,1 bis 2 µg/ml erreicht wird. Bevorzugt ist eine Konzentration von 0,8 bis 1,2 µg/ml.

Für spezielle Anwendungen, wie z.B. für die Hämodialyse bzw. bei der extrakorporalen Blutzirkulation, z.B. Bypass-Operationen, ist es bevorzugt, das Mittel an Trägern und/oder Polymere zu fixieren, die dann mit Blut in Berührung kommen, z.B. in Behältern und Apparaten. Das Mittel kann auch an lipophile Substanzen adsorbiert oder gekoppelt sein, um damit Oberflächen zu behandeln.

Das erfindungsgemäße Mittel ist insbesonders allgemein zur Behandlung entzündlicher Erkrankungen, die mit Leukozytenaktivierung verbunden sind, zur Behandlung des septischen Schocks oder zur Frühbehandlung bei Polytrauma zur Vermeidung eines posttraumatischen Schocks einzusetzen.

Die Erfindung wird durch die nachstehenden Beispiele näher erläutert:

### 1. Beispiel

### Fixierung von Angiogenin bzw. Angiogeninpeptid

Verfahren, die zur kovalenten Fixierung von Proteinen und Peptiden an polymeren Trägern geeignet sind, können auch dazu dienen, Angiogenin und Angiogeninfragmente bzw. synthetische Peptide oder entsprechende Peptidomimetika an Membranen, z.B. Dialysemembranen, zu koppeln, z.B.:

### Agarose-Gele

mit aktiven Carboxylestern über α- bzw. ε-(Aminogruppe zur Amidbindung (entsprechend Affigel 10, Fa. BioRad),
mit primärem Amin über Carboxylgruppe zum Amid mittels Kopplungsreagenz, z.B. wasserlöslischem Cyclohexylcarbodiimid (entsprechend Affigel 102)
mit Sulfhydrylgruppen über Disulfidbildung zu Cysteinresten oder über Thioester (entsprechend Affigel 401)
oder

### Polyacrylamid-Träger

mit aktiver Hydrazidgruppe über die Carboxylgruppe (entsprechend Hydrazide BioGel P),
mit primärem Amin über die Carboxylgruppe (entsprechend Affigel 701),
mit Carboxylgruppe über α- bzw. ε-Aminogruppe mittels Kopplungsreagenz, z.B. Carbodiimiden
oder

### Polystyrol Gele

mit Chlormethylgruppe zur Reaktion mit α- bzw. ε-Aminogruppe (entsprechend Bio-Beads SX1)
oder

### Pericellulose

mit ONB-Carbonat* als reaktive Gruppe zur Reaktion mit α- bzw. ε-Aminogruppen (entsprechend Eurocell ONB-Carbonat der Fa. Knauer)

* 5-Norbornen-2,3-dicarboxylimidyl-chlorkohlensäureester (CI-CO-ONB)

### 2. Beispiel

### Mittel zur Hemmung der Degranulation bei extrakorporaler Zirkulation

Das Mittel wird in steriler, physiologischer Kochsalzlösung gelöst, gegebenenfalls unter Zusatz von Humanalbumin und dem Blutstrom zugemischt, so daß maximal eine Konzentration von 0,1 - 1 µg/ml in dem zirkulierenden Blutstrom entsteht. Der maximale Bedarf pro Patient liegt damit in der Größenordnung von 0,5 - 5 mg für Hämodialyse bzw. extrakorporalen Bypass.

### Mittel zur Behandlung der Lungenentzündung

Das Mittel wird gelöst in steriler, physiologischer Kochsalzlösung (Konzentration 0,1 - 1 µg/ml), gegebenenfalls versetzt mit einem natürlichen Netzmittel, z.B. humanem, rekombinanten Lungensurfaktant, und als Aerosol inhaliert.

## Patentansprüche

1. Verwendung von Angiogenin und/oder α₁-Microglobulin und/oder Komplementfaktor D und/oder deren Fragmente bzw. Peptide mit den biologisch wirksamen Sequenzbereichen dieser Peptide und/oder Peptidomimetika zur Herstellung eines therapeutischen Mittels zur Bekämpfung von Entzündungsprozessen, zur Behandlung des septischen Schocks oder zur Frühbehandlung bei Polytrauma zur Vermeidung eines posttraumatischen Schocks.

2. Therapeutisches Mittel zur Verwendung nach Anspruch 1,
dadurch **gekennzeichnet,**
daß es α₁-Microglobulin und/oder Fragmente bzw. Peptide mit dem biologisch wirksamen Sequenzbereich dieser Peptide und übliche Träger- und Zusatzstoffe enthält.

3. Therapeutisches Mittel zur Verwendung nach Anspruch 1,
dadurch **gekennzeichnet,**
daß es Komplementfaktor D und/oder Fragmente bzw. Peptide mit den biologisch wirksamen Sequenzbereichen dieser Peptide und übliche Träger- und Zusatzstoffe enthält.

4. Therapeutisches Mittel zur Verwendung nach Anspruch 1 oder 2,
dadurch **gekennzeichnet,**
daß es Angiogenin und α₁-Microglobulin und/oder Fragemente bzw. Peptide mit den biologisch wirksamen Sequenzbereichen dieser Peptide und übliche Träger- und Zusatzstoffe enthält.

5. Therapeutisches Mittel zur Verwendung nach Anspruch 1,
dadurch **gekennzeichnet,**
daß es als Wirkstoff Angiogenin und Komplementfaktor D und/oder Fragmente bzw. Peptide mit den biologisch wirksamen Sequenzbereichen dieser Peptide und übliche Träger- und Zusatzstoffe enthält.

6. Therapeutisches Mittel zur Verwendung nach Anspruch 1,
dadurch **gekennzeichnet,**
daß es Komplementfaktor D und α₁-Microglobulin und/oder Fragmente bzw. Peptide mit den biologisch wirksamen Sequenzbereichen dieser Peptide und übliche Träger- und Zusatzstoffe enthält.

7. Therapeutisches Mittel zur Verwendung nach Anspruch 1,
dadurch **gekennzeichnet,**
daß es als Wirkstoff Angiogenin und α₁-Microglobulin und Komplementfaktor D und/oder Fragmente bzw. Peptide mit den biologisch wirksamen Sequenzbereichen dieser Peptide und übliche Träger- und Zusatzstoffe enthält.

8. Therapeutisches Mittel nach Anspruch 2 bis 7,
dadurch **gekennzeichnet,**
daß es ein Fragment von Angiogenin in der folgenden Sequenz 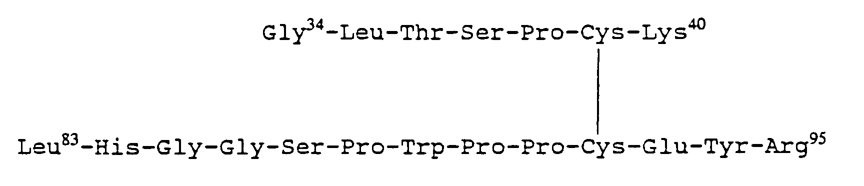
Gly³⁴-Leu-Thr-Ser-Pro-Cys-Lys⁴⁰
Leu⁸³-His-Gly-Gly-Ser-Pro-Trp-Pro-Pro-Cys-Glu-Tyr-Arg⁹⁵
 enthält.

9. Therapeutisches Mittel nach Anspruch 2 bis 8,
dadurch **gekennzeichnet,**
daß es ein synthetisches Peptid von Angiogenin der folgenden Sequenz,
Leu⁸³-His-Gly-Gly-Ser-Pro-Trp-Pro-Pro-Ser-Glu-Tyr-Arg⁹⁵
enthält.

10. Therapeutisches Mittel nach Anspruch 4, 5 oder 7 bis 9,
dadurch **gekennzeichnet,**
daß es das 123-Aminosäure umfassende Angiogenin, das aus Körperflüssigkeiten oder als rekombinantes Protein gewonnen wird, enthält.

11. Therapeutisches Mittel nach Anspruch 4 oder 6 bis 9,
dadurch **gekennzeichnet,**
daß es humanes α₁-Microglobulin mit einem mittleren Molekulargewicht von ca. 30.000, das aus Körperflüssigkeiten oder als rekombinantes Protein gewonnen wird, enthält.

12. Therapeutisches Mittel nach Anspruch 2 bis 11,
dadurch **gekennzeichnet,**
daß der Wirkstoff in einer Konzentration im Mittel vorliegt, durch die eine maximale Konzentration von 0,1 bis 2 µg/ml im Blut resultiert.

13. Therapeutisches Mittel nach Anspruch 12,
dadurch **gekennzeichnet,**
daß der Wirkstoff in einer entsprechenden Konzentration vorliegt, durch die eine maximale Konzentration von 0,3 bis 1,2 µg/ml im Blut resultiert.

14. Therapeutisches Mittel nach Anspruch 2 bis 11,
dadurch **gekennzeichnet,**
daß es an Träger und/oder Polymere fixiert ist, die mit Blut in Berührung kommen.

15. Therapeutisches Mittel nach Anspruch 2 bis 11,
dadurch **gekennzeichnet,**
daß es als Lösung für die intravenöse Behandlung oder als Spray oder als feste Formulierung vorliegt.

## Claims

1. The use of angiogenin and/or α₁-micrcglobulin and/or complement factor D and/or their fragments resp peptides with the biologically effeotive sequential ranges of said peptides and/or peptidomimetics for preparing a therapeutic agent for fighting inflammation processes, for treating the septic shock or for early treatment in case of multiple trauma so as to avoid a post-traumatic shock.

2. Therapeutic agent for use according to Claim 1,
**characterised** in that said agent contains α₁-microglobulin and/or fragments resp peptides with the biologically effective sequential range of said peptides and usual carrier and additive substances.

3. Therapeutic agent for use according to Claim 1,
**characterised** in that said agent contains complement factor D and/or fragments resp peptides with the biologically effective sequential ranges of said peptides and usual carrier and additive substances.

4. Therapeutic agent for use according to Claim 1 or 2,
**characterised** in that said agent contains angiogenin and α₁-microglobulin and/or fragments resp peptides with the biologically effective sequential ranges of said peptides and usual carrier and additive substances.

5. Therapeutic agent for use according to Claim 1,
**characterised** in that said agent contains, as active substance, angiogenin and complement factor D and/or fragments resp peptides with the biologically effective sequential ranges of said peptides and usual carrier and additive substances.

6. Therapeutic agent for use according to Claim 1,
**characterised** in that said agent contains complement factor D and α₁-microglobulin and/or fragments resp peptides with the biologically effective sequential ranges of said peptides and usual carrier and additive substances.

7. Therapeutic agent for use according to Claim 1,
**characterised** in that said agent contains, as active substance, angiogenin and α₁-microglobulin and complement factor D and/or fragments resp peptides with the biologically effective sequential ranges of said peptides and usual carrier and additive substances.

8. Therapeutic agent according to Claims 2 to 7,
**characterised** in that said agent contains a fragment of angiogenin in the following sequence:

Gly34-Leu-Thr-Ser-Pro-Cys-Lys40
|
Leu83-His-Gly-Gly-Ser-Pro-Trp-Pro-Pro-Cys-Glu-Tyr-Arg95

9. Therapeutic agent according to Claims 2 to 8,
**characterised** in that said agent contains a synthetic peptide of angiogenin of the following sequence:
Leu⁸³-His-Gly-Gly-Ser-Pro-Trp-Pro-Pro-Ser-Glu-Tyr-Arg⁹⁵

10. Therapeutic agent according to Claims 4, 5 or 7 to 9,
**characterised** in that said agent contains that angiogenin which comprises 123-amino acid and which is gained from body fluids or in the form of recombinant protein.

11. Therapeutic agent according to Claims 4 or 6 to 9,
**characterised** in that said agent contains human α₁-microglobulin having a mean molecular weight of ca. 30,000 and being gained from body fluids or in the form of recombinant protein.

12. Therapeutic agent according to Claims 2 to 11,
**characterised** in that the active substance is present in a concentration in agent, by which concentration a maximum concentration of from 0.1 to 2 µg/ml in blood results.

13. Therapeutic agent according to Claim 12,
**characterised** in that the active substance is present in an appropriate concentration by which a maximum concentration of from 0.3 to 1.2 µg/ml in blood results.

14. Therapeutic agent according to Claims 2 to 11,
**characterised** in that said agent is fixed to carriers and/or polymers that come into contact with blood.

15. Therapeutic agent according to Claims 2 to 11,
**characterised** in that said agent is present in the form of a solution for intravenous treatment or in the form of a spray or in the form of a solid formulation.

## Revendications

1. Utilisation de l'Angiogénine et/ou de l'α₁-Microglobuline et/ou du facteur complémentaire D et/ou de leurs fragments ou peptides avec leurs zones de séquences biolgiquement actives et/ou des peptidomimétiques pour la fabrication d'un agent thérapeutique contre les processus inflammatoires, pour le traitement du choc septique ou le traitement précoce en cas de polytraumatisme afin d'éviter un choc posttraumatique.

2. Agent thérapeutique destiné à l'utilisation répondant à la revendication 1 et **caractérisé par**
la présence dans sa composition de l'α₁-Microglobuline et/ou des fragments ou peptides avec leurs zones de séquences biologiquement actives ainsi que les produits de support et additifs habituels.

3. Agent thérapeutique destiné à l'utilisation répondant à la revendication 1 et **caractérisé par**
la présence dans sa composition du facteur complémentaire D et/ou des fragments ou peptides avec leurs zones de séquences biologiquement actives ainsi que les produits de support et additifs habituels.

4. Agent thérapeutique destiné à l'utilisation répondant aux revendications 1 ou 2 et **caractérisé par**
la présence dans sa composition de l'Angiogénine et de l'α₁-Microglobuline et/ou des fragments ou peptides avec leurs zones de séquences biologiquement actives ainsi que les produits de support et additifs habituels.

5. Agent thérapeutique destiné à l'utilisation répondant à la revendication 1 et **caractérisé par**
la présence dans sa composition comme agent actif de l'Angiogénine et du facteur complémentaire D et/ou des fragments ou peptides avec leurs zones de séquences biologiquement actives ainsi que les produits de support et additifs habituels.

6. Agent thérapeutique destiné à l'utilisation répondant à la revendication 1 et **caractérisé par**
la présence dans sa composition du facteur complémentaire D et de l'a₁-Microglobuline et/ou des fragments ou peptides avec leurs zones de séquences biologiquement actives ainsi que les produits de support et additifs habituels.

7. Agent thérapeutique destiné à l'utilisation répondant à la revendication 1 et **caractérisé par**
la présence dans sa composition comme agent actif de l'Angiogénine et de l'α₁-Microglobuline et du facteur complémentaire D et/ou des fragments ou peptides avec leurs zones de séquences biologiquement actives ainsi que les produits de support et additifs habituels.

8. Agent thérapeutique répondant aux revendications 2 à 7 et **caractérisé par**
la présence dans sa composition d'un fragment de l'Angiogénine de la séquence suivante

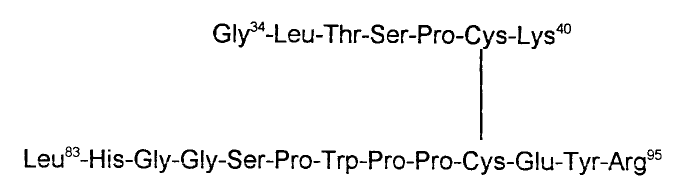
Gly34-Leu-Thr-Ser-Pro-Cys-Lys40
Leu83-His-Gly-Gly-Ser-Pro-Trp-Pro-Pro-Cys-Glu-Tyr-Arg95

9. Agent thérapeutique répondant aux revendications 2 à 8 et **caractérisé par**
la présence dans sa composition d'un peptide synthétique de l'Angiogénine de la séquence suivante
Leu⁸³-His-Gly-Gly-Ser-Pro-Trp-Pro-Pro-Ser-Glu-Tyr-Arg⁹⁵

10. Agent thérapeutique répondant aux revendications 4, 5 ou 7 à 9 et **caractérisé par**
la présence dans sa composition de l'Angiogénine comprenant 123 acides aminés, obtenue à partir des humeurs corporelles ou comme protéine de recombinaison.

11. Agent thérapeutique répondant aux revendications 4 ou 6 à 9 et **caractérisé par**
la présence dans sa composition de l'α₁-Microglobuline humaine avec un poids moléculaire moyen d'environ 30 000 obtenue à partir des humeurs corporelles ou comme protéine de recombinaison.

12. Agent thérapeutique répondant aux revendications 2 à 11 et **caractérisé par**
une concentration en produit actif entraînant une concentration sanguine maximale de 0,1 à 2 µg/ml.

13. Agent thérapeutique répondant à la revendication 12 et **caractérisé par**
une concentration en produit actif adéquate telle qu'elle entraîne une concentration sanguine maximale de 0,3 à 1,2 µg/ml.

14. Agent thérapeutique répondant aux revendications 2 à 11 et **caractérisé par**
sa fixation à des supports et/ou polymères qui entrent en contact avec le sang.

15. Agent thérapeutique répondant aux revendications 2 à 11 **caractérisé par**
sa présentation sous forme de solution pour le traitement intraveineux ou de spray ou de formulation solide.
